# EUROPÄISCHE PATENTANMELDUNG

(11) **EP 0 694 533 A1**
(43) Veröffentlichungstag der Anmeldung: **31.01.1996**
(21) Anmeldenummer: 95110972.7
(22) Anmeldetag: 13.07.1995
(51) Int. Cl.: C07D 209/48, C07B 43/04, C07D 401/04, A61K 31/47

(54) **4-Amino-3-hydroxy-phthalimidin, sowie ein Verfahren zu seiner Herstellung**

(30) Priorität: 26.07.1994 DE 4426374
(71) Anmelder: BAYER AG, D-51368 Leverkusen (DE)
(72) Erfinder: Stoltefuss, Jürgen, Dipl.-Ing., D-42781 Haan (DE); Negele, Michael, Dr., D-42697 Solingen (DE); Dürholz, Friedrich, Dr., D-42897 Remscheid (DE)

(57) **Zusammenfassung**

Die vorliegende Erfindung betrifft 4-Amino-3-hydroxy-phthalimidin der Formel (I) 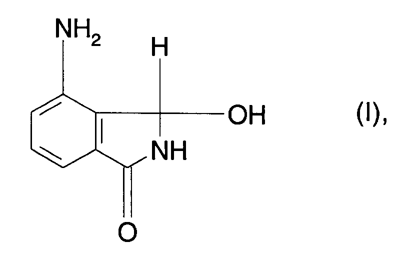
ein wichtiges Zwischenprodukt zur Synthese von 3-substituierten 5-Chinolincarbonsäureamiden sowie ein Verfahren zu seiner Herstellung.

## Beschreibung

Die vorliegende Erfindung betrifft 4-amino-3-hydroxyphthalimidin, ein wichtiges Zwischenprodukt zur Synthese von 3-substituierten 5-Chinolin-carbonsäureamiden sowie ein Verfahren zu seiner Herstellung.

Aus der Publikation J. Chem. Pharm. Bull. 26, 530-538 (1978) ist 3-Hydroxy-4-nitrophthalimidin bereits bekannt.

Gegenstand der vorliegenden Erfindung ist die neue Verbindung 4-Amino-3-hydroxyphthalimidin der Formel (I). 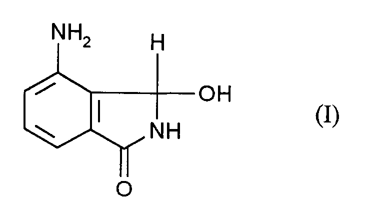
Die Erfindung betrifft weiterhin ein Verfahren zur Herstellung der Verbindung der Formel (I), dadurch gekennzeichnet, daß man
4-Nitro-3-hydroxyphthalimidin der Formel (II) 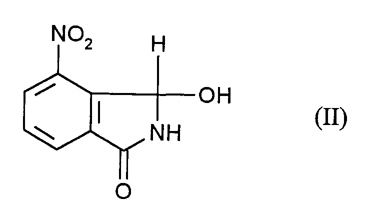
in inerten Lösemitteln in Anwesenheit eines Katalysators, in Lösung oder in Suspension, nach üblichen Methoden hydrierend reduziert.

Das Verfahren kann durch folgendes Formelschema erläutert werden: 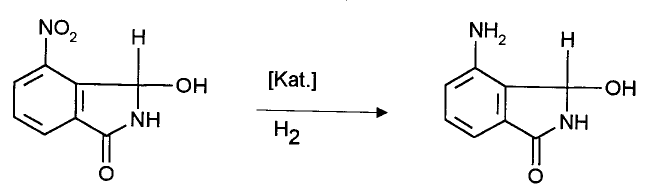
Als Lösemittel eignen sich für die Hydrierung Wasser und alle organischen Lösemittel, die sich unter den Reaktionsbedingungen nicht verändern. Hierzu gehören Alkohole wie Methanol, Ethanol, Propanol oder Isopropanol, oder Ether wie Diethylether, Dioxan, Tetrahydrofüran, Glykoldimethylether oder Diethylenglykoldimethylether, oder Amide wie Hexamethylphosphorsäuretriamid oder Dimethylformamid, oder Essigsäure, sowie Methylenchlorid, Tetrachlorkohlenstoff oder Toluol. Ebenso ist es möglich, Gemische der genannten Lösemittel zu verwenden. Auch können Gemische von beispielsweise Methanol, Ethanol, Propanol oder Tetrahydrofuran und deren Mischungen mit Wasser eingesetzt werden. Bevorzugt sind in diesem Fall Mischungen von Methanol mit Wasser.

Die Hydrierung kann bei Normaldruck oder bei erhöhtem Druck, beispielsweise von 0,5 bis 100 bar, bevorzugt bei 5 - 50 bar, besonders bevorzugt 8 - 12 bar, durchgeführt werden.

Geeignete Katalysatoren für die katalytische Hydrierung mit Wasserstoff sind beispielsweise solche, die aus Metallen und/oder Verbindungen von Elementen der 8. Nebengruppe des periodischen Systems der Elemente bestehen oder diese enthalten. Dabei sind die Metalle Ruthenium, Rhodium, Palladium, Platin, Kobalt und Nickel und deren Verbindungen bevorzugt. Derartige Metalle und Metallverbindungen können auch auf Trägermaterialien aufgebracht sein. Metallische Katalysatoren können auch als Skelettkatalysatoren vom Raney-Typ eingesetzt werden.

Geeignete Katalysatormengen sind beispielsweise solche von 0,00001 mol bis 1 mol, bevorzugt 0,0001 bis 0,1 mol, bezogen auf 1 mol der Formel (II).

Besonders bevorzugt sind Palladium-Katalysatoren auf Trägern wie Pd/C, Pd/BaSO₄ , Pd/Al₂O₃ oder Raney-Nickel.

Für den Reaktionsverlauf erweist sich gegebenenfalls der Zusatz von basischen Salzen als vorteilhaft. Dazu gehören Alkali- oder Erdalkaliacetate wie beispielsweise Natrium- oder Kaliumacetat, oder Alkalicarbonate oder Alkalihydrogencarbonate wie beispielsweise Natrium- oder Kaliumcarbonat oder -hydrogencarbonat.

Die Verbindung der Formel (II) ist bekannt [vgl. T. Watanabe et al., Chem. Pharm. Bull, 20(10), 2123-2127(1972)].

Das vorstehende Herstellungsverfahren kann sowohl in Batch-Fahrweise als auch halbkontinuierlich durchgeführt werden.

Das vorstehende Herstellungsverfahren ist lediglich zur Verdeutlichung angegeben. Die Herstellung der erfindungsgemäßen Verbindung der Formel (I) ist nicht auf dieses Verfahren beschränkt.

Die erfindungsgemäße Verbindung ist ein wertvolles Ausgangs- bzw. Zwischenprodukt zur Herstellung von 3-substituierten Chinolinaldehyden, die als Vorstufen für 1,4-Dihydropyridine von Bedeutung sind. Sie lassen sich z.B. mit β-Ketoestem und Enaminen zu pharmakologisch interessanten Dihydropyridin-Wirkstoffen zyklisieren.

### Herstellungsbeispiele

### Beispiel 1

### 4-Amino-3-hydroxyphthalimidin

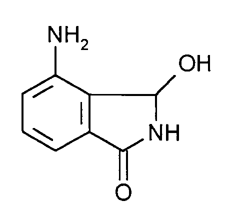

### Vorschrift A:

20 g (104 mmol) 3-Hydroxy-4-nitro-phthalimidin werden in 1 l Methanol warm gelöst und mit 2,5 g Pd/BaSO₄ bei 3,5 bar hydriert. Dabei steigt die Temperatur der Reaktion bis 55°C. Nach 5 Minuten war die Reaktion beendet, es wird bei ca. 50°C über Celite abgesaugt und mit Methanol gewaschen. Das Filtrat wird fast bis zur Trockene eingeengt, abgesaugt und mit kaltem Methanol gewaschen. Man erhält 14,6 g (86,5% d.Th.) leicht gelber Kristalle vom Schmelzpunkt ab 260°C (Zers.).
Aus der Mutterlauge werden durch Einengen und Absaugen mit Essigester noch 1,4 g (8,3 % d.Th.) Kristalle vom Schmelzpunkt ab 260°C (Zers.) erhalten.

### Vorschrift B:

582,4 g (3 mol) 3-Hydroxy-4-nitro-phthalimidin und 37 g Natriumacetat werden in 3,6 l Methanol in einem 10 l-V4A-Rührwerksautoklav bei ca. 40°C und einem Wasserstoffdruck von 6-10 bar an 30 g Palladium/Kohle (5% Palladium) hydriert. Die Reaktionszeit beträgt 2 Stunden. Die Suspension wird abschließend abfiltriert, das Rohprodukt mit Katalysator getrocknet und weiterverarbeitet.
Ausbeute: 395,7 g (80,3% d.Th.)

### Vorschrift C:

582,4 g (3 mol) 3-Hydroxy-4-nitro-phthalimidin werden in 3,5 l Methanol in einem 10 l VA-Rührwerksautoklav bei ca. 30°C und einem Wasserstoffdruck von 30 bar an 30 g Raney-Nickel hydriert. Die Reaktionszeit beträgt 8 Stunden. Die Suspension wird abfiltriert, mit Wasser gewaschen und das katalytische Rohprodukt wasserfeucht weiterverarbeitet.
Ausbeute: 98,5% d.Th. (lt. HPLC; Gew.-%-Auswertung)

### Vorschrift D:

110 g 3-Hydroxy-4-nitrophthalimidin (ca. 87%ige Rohware) werden in 600 ml Methanol in einem 1,3 l VA-Autoklaven mit Ankerblatt-Rührer mit Zusatz von ca. 5 g (5%) Pd/Kohle und 10 g NaHCO₃ hydriert. Die Reaktionszeit beträgt 1 h. Dann wird 1 h bei konstantem H₂-Druck nachhydriert. Durch Verrühren mit 2,3 l Methanol bei 60°C wird das Hydrierungsprodukt weitesgehend gelöst. Es wird filtiert und am Rotationsverdampfer eingeengt. Man erhält 76 g (91% d.Th) Kristalle.

### Vorschrift E:

In Analogie zur Vorschrift der Variante D erfolgt die Umsetzung bei 50 bar H₂ und an 1,7 g (5%) Pd/Kohle unter Zusatz von 20g NaHCO₃. Man erhält 73,2 g (88,4% d.Th.) eines leicht gelblichen Festprodukts.

## Patentansprüche

1. 4-Amino-3-hydroxyphthalimidin der Formel (I)

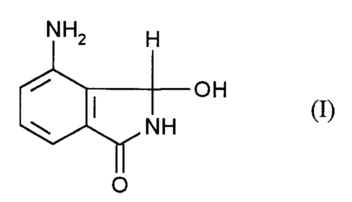

2. Verfahren zur Herstellung der Verbindung der Formel (I) gemäß Anspruch 1, dadurch gekennzeichnet, daß man 4-Nitro-3-hydroxyphthalimidin der Formel (II) 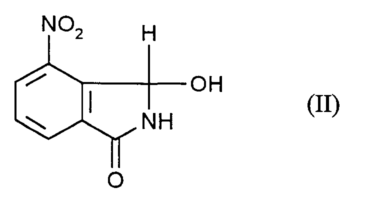 in inerten Lösemitteln in Anwesenheit eines Katalysators in Lösung oder in Suspension hydrierend reduziert.

3. Verfahren gemäß Anspruch 2, dadurch gekennzeichnet, daß man die Hydrierung bei 0,5-100 bar und gegebenenfalls in Gegenwart von basischen Salzen durchführt.

4. Verfahren gemäß Anspruch 2, dadurch gekennzeichnet, daß man Katalysatoren verwendet, die auf Trägermaterialien aufgebracht sind.

5. Verfahren gemäß Anspruch 2, dadurch gekennzeichnet, daß man Palladiumkatalysatoren verwendet.

6. Verwendung der Verbindung der Formel (I) gemäß Anspruch 1 als Ausgangsprodukt bei der Herstellung von 3-substituierten Chinolinaldehyden.
